(19) Europäisches Patentamt
European Patent Office
Office européen des brevets

(11) Veröffentlichungsnummer: **0 330 058 B1**

(12) **EUROPÄISCHE PATENTSCHRIFT**

(45) Veröffentlichungstag der Patentschrift: **02.10.91 Patentblatt 91/40**

(51) Int. Cl.[5]: **C07F 7/18**, C07F 7/12, C07C 29/40, C07C 31/38

(21) Anmeldenummer: **89102540.5**

(22) Anmeldetag: **15.02.89**

(54) **Verfahren zur Herstellung von Perfluoralkylgruppen enthaltenden Silyläthern und Alkoholen.**

(30) Priorität: **23.02.88 DE 3805534**

(43) Veröffentlichungstag der Anmeldung: **30.08.89 Patentblatt 89/35**

(45) Bekanntmachung des Hinweises auf die Patenterteilung: **02.10.91 Patentblatt 91/40**

(84) Benannte Vertragsstaaten: **CH DE FR GB IT LI NL**

(56) Entgegenhaltungen:
**CHEMICAL ABSTRACTS, Band 93, Nr. 5, 04.Aug 1980, Seite 289F, Spalte 1, Zeilen 25-27, Z'fassung Nr. 46773c, Columbus, Ohio, USA. B.GHOSE: "The preparation of some hepta-fluoro-n- and isopropyl derivatives of phosphorus and silicon"**

(56) Entgegenhaltungen:
**JOURNAL OF ORGANOMETALLIC CHEMISTRY, Band 292, 1985, Seiten 145-149, Lausanne, CH; O A VYAZANKINA et al.: "Cyanide-ion-catalyzed reaction of pentafluorophenyltrimethylsilane with substituted acetophenones".**
**J. Chem. Soc.; Chem. Commun. 1987, pp 642,3.**

(73) Patentinhaber: **HOECHST AKTIENGESELLSCHAFT**
**Postfach 80 03 20**
**W-6230 Frankfurt am Main 80 (DE)**

(72) Erfinder: **Kruse, Alfred, Dr.**
**Hornauer Strasse 199**
**W-6233 Kelkheim (Taunus) (DE)**
Erfinder: **Siegemund, Günter, Dr.**
**Frankfurter Strasse 21**
**W-6238 Hofheim am Taunus (DE)**
Erfinder: **Schumann, Axel**
**Willi-Haas-Strasse 34**
**W-5305 Alfter 3 (DE)**
Erfinder: **Ruppert, Ingo Walter, Dr.**
**verstorben (DE)**

EP 0 330 058 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Gegenstand der Erfindung ist ein Verfahren zur Herstellung von Perfluoralkylverbindungen unter Verwendung von Perfluoralkyl-trimethylsilanen der allgemeinen Formel

$$(CH_3)_3Si\text{-}C_nF_{2n+1} \quad (I)$$

Verfahren zur Perfluoralkylierung von Carbonylverbindungen sind von großem Interesse. Zur Anwendung kommen dafür Perfluoralkylmetallverbindungen unedler Metalle, die gewöhnlich aus entsprechenden Perfluoralkyljodiden und einem Metall wie Na, Li, Mg, Zn, Cd hergestellt werden. Hier sind die in der Regel aufwendige Herstellung und die Labilität der zunächst herzustellenden Perfluoralkylmetallverbindung von Nachteil, was u.a. darin zum Ausdruck kommt, daß die publizierten Ergebnisse nur schlecht reproduzierbar sind. (Tetrahedron Letters 26, 5243-6 (1985) speziell Seite 5245, Fußnote 4 und dort zitierte Literatur).

Weitere Nachteile sind z.B., daß die Umsetzung bei tiefen Temperaturen von −78°C bis −100°C sowie in hoher Verdünnung (Tetrahedron Letters 26, 5243-6) durchgeführt werden muß, daß die Anwendung von Ultraschall erforderlich ist (Chem. Letters 1981, 1679-80) und daß das Verfahren auf wenige Substrate beschränkt ist, d.h. daß keine oder nur geringe Ausbeuten bei der Umsetzung von enolisierbaren Carbonylverbindungen erhalten werden (J. Chem. Soc., Chem. Commun. 1987, 642-3).

Die Reaktion von Carbonylverbindungen mit Pentafluorphenyl-trimethylsilanen ist bekannt. Der Perfluorarylrest läßt sich jedoch nur bei der Umsetzung mit nicht enolisierbaren Carbonylverbindungen wie Benzahldehyd oder Trifluoracetophenon auf die Carbonylverbindung übertragen ; im anderen Fall wird die Carbonylverbindung lediglich in den entsprechenden Trimethylsilylenoläther übergeführt, und eine Übertragung des Perfluorarylrestes findet nicht statt (J. Organometallic Chem. 292, 145-9 (1985) ; Chem. Letters 1972, 435-6).

Es wurde nun überraschend gefunden, daß sich durch Umsetzung von Perfluoralkyl-trimethylsilanen ein Perfluoralkylrest auch von anderen als Organometallverbindungen übertragen läßt. Besonders überraschend war, daß diese Reaktion im Gegensatz zu der vorher beschriebenen auch beim Einsatz enolisierbarer Carbonylverbindungen das gesuchte Perfluoralkylierungsprodukt ergibt.

Die Erfindung bezieht sich auf ein einfaches, einstufiges Verfahren zur Übertragung von Perfluoralkylresten $C_nF_{2n+1}$ auf Carbonylverbindungen durch deren Umsetzung mit Perfluoralkyl-trimethylsilanen der allgemeinen Formel

$$(CH_3)_3Si\text{-}C_nF_{2n+1} \quad (I).$$

Dabei werden in einem Schritt Silyläther der allgemeinen Formel III (siehe Patentanspruch 1) gebildet.

Das erfindungsgemäße Verfahren hat insbesondere den Vorteil der Einfachheit sowie den einer großen Anwendungsbreite in der Art und Struktur der eingesetzten Carbonylverbindung. So werden auch in den Fällen gute Ausbeuten erzielt, bei denen bekannte Verfahren nur geringe Ausbeuten liefern, wie es beispielsweise bei der Umsetzung von Carbonylverbindungen mit Zink und Trifluorbrommethan der Fall ist, wo Ausbeuten von 20-65% erzielt wurden (J. Chem. Soc., Chem. Commun 1987, 642-3).

Die Einfachheit des Verfahrens ist schon daraus zu erkennen, daß es ausreicht, wenn die beiden Komponenten in Gegenwart eines Katalysators einige Zeit bei Raumtemperatur in einem inerten Lösungsmittel gerührt werden. Im allgemeinen wird die Umsetzung jedoch bei 0 bis 100°C, vorzugsweise bei 20 bis 60°C durchgeführt. Das Reaktionsprodukt läßt sich leicht in Form des gebildeten Silyläthers isolieren. Aus diesen Äthern können die zugrundeliegenden Alkohole, die bekannt sind, leicht durch hydrolytische Abspaltung der Trimethylsilylgruppe erhalten werden.

In dem erfindungsgemäßen Verfahren können als Perfluoralkyl-trimethylsilane solche der allgemeinen Formel $(CH_3)_3Si\text{-}C_nF_{2n+1}$ mit n = 1 bis n = 6 eingesetzt werden. Bevorzugt ist n = 1 bis 4. Die Reste $C_nF_{2n+1}$ können geradkettig oder verzweigt sein.

Als Carbonylverbindungen werden Verbindungen der allgemeinen Formel

$$R^1\text{-CO-}R^2 \quad (II)$$

eingesetzt. Die Reste $R^1$ und $R^2$ können eine Vielzahl von Bedeutungen haben. $R^1$ kann einen beliebigen Kohlenwasserstoffrest, wie Alkyl, Alkenyl, Cycloalkyl oder Aryl, oder Wasserstoff bedeuten ; $R^2$ kann ebenfalls einen beliebigen Kohlenwasserstoffrest, wie Alkyl, Alkenyl, Cycloalkyl oder Aryl bedeuten, jedoch auch Perfluoralkyl oder Perfluoraryl. $R^1$ und $R^2$ zusammen können auch Bestandteil eines alicyclischen Ringsystems sein, also zusammen mit dem Kohlenstoffatom der Carbonylgruppe z.B. den Cyclopentylen- oder Cyclohexylenrest bedeuten, die auch durch Alkyl wie Methyl, Äthyl oder Isopropyl substituiert sein können. $R^1$ und $R^2$

haben z.B. jeweils 1 bis 10, insbesondere 1 bis 6 C-Atome. Geeignete Reste sind z.B. die Methyl-, Äthyl-, n- und Isopropyl und die verschiedenen Butyl-, Pentyl-, Hexyl-, Octyl- und Decylreste, ferner der Vinyl- oder Allylrest oder der Rest $C_6H_5CH{=}CH$— und der Phenyl- oder Naphthylrest, wobei der Phenylrest noch durch Alkylreste mit insgesamt bis zu 4 C-Atomen substituiert sein kann, also z.B. ein Tolyl-, Xylyl-, Cumyl-, Cymyl- oder t-Butylrest oder ein Hydrierungsprodukt davon ist.

Als Katalysator werden salzartige Fluoride verwendet, die in dem Reaktionsmedium eine gewisse Löslichkeit haben. Bevorzugt werden wegen der leichten Zugänglichkeit Alkalifluoride und Alkalibifluoride, wobei der Begriff Alkali Ammoniumbasen einschließt. Insbesondere eignen sich Kaliumfluorid und Cäsiumfluorid. Im allgemeinen wird der Katalysator in einer Menge von 2 bis 30 Mol-%, vorzugsweise von 10 bis 25 Mol-%, bezogen auf die Carbonylverbindung, verwendet.

Zweckmäßig wird das vorliegende Verfahren unter wasserfreien Bedingungen in Gegenwart eines gegenüber den Reaktions- teilnehmern unter den Reaktionsbedingungen inerten Lösungs- oder Verdünnungsmittels durchgeführt. Geeignet sind aprotische, polare Lösungsmittel, die ein gewisses Lösungsvermögen für den Katalysator besitzen. Besonders geeignet sind Äther wie Tetrahydrofuran oder Polyäther der allgemeinen Formel $CH_3\text{-}O\text{-}[CH_2\text{-}Alk\text{-}O]_n\text{-}CH_3$ mit n = 1 bis n = 4, worin Alk $CH\text{-}CH_3$ oder vorzugsweise $CH_2$ bedeutet, sowie Dimethylformamid.

Das erfindungsgemäße Verfahren kann beispielsweise so durchgeführt werden, daß man Lösungsmittel, Carbonylverbindung und Katalysator vorlegt und das Silan zudosiert. Das Silan kann in einer zur Carbonylverbindung äquivalenten Menge oder in einem Überschuß, z.B. von bis zu 25 Mol-%, eingesetzt werden. Es ist von Vorteil, während der gesamten Dauer der Reaktion, z.B. durch Rühren, für eine gute Durchmischung des Ansatzes zu sorgen. Die Umsetzung wird im allgemeinen bei Normaldruck ausgeführt ; jedoch kann man auch bei erhöhtem oder vermindertem Druck arbeiten, wenn hiermit im allgemeinen auch kein Vorteil verbunden ist.

Die Aufarbeitung des Reaktionsgemisches läßt sich auf verschiedene Weise vornehmen. Vorteilhaft erfolgt sie durch destillative Trennung der Komponenten. Verwendet man jedoch ein wasserlösliches Lösungsmittel wie Tetraäthylenglykol-dimethyläther, kann die Aufarbeitung in einfacher Weise durch eine Extraktion mit Wasser erfolgen. Das resultierende, in Wasser unlösliche Rohrprodukt kann dann bei Bedarf destillativ gereinigt werden.

Die Perfluoralkyl-trimethylsilane sind im allgemeinen bekannt. Lediglich das Pentafluoräthyl-trimethylsilan ist neu. Die Herstellung gelingt in hohen Ausbeuten durch Übertragung eine Perfluoräthylgruppe auf Chlor-trimethylsilan $Cl\text{-}Si(CH_3)_3$ in einer von der bekannten Herstellung von Trifluormethyl-trimethylsilan (Tetrahedron Letters 25, 2195-8 (1984) verschiedenen Weise. Dabei wird Chlortrimethylsilan mit Pentafluoräthyljodid $CF_3CF_2J$ und Phosphorigsäuretrisdialkylamiden (in anderen Worten Tris(dialkylamino)phosphanen) der allgemeinen Formel $P(NAlkyl)_2)_3$ (V), umgesetzt, im allgemeinen bei Temperaturen zwischen −80°C und 0°C, vorzugsweise zwischen −30°C und −10°C. Die bekannte Umsetzung von Chlortrimethylsilan erfolgt mit Trifluormethylbromid, und zwar bei "möglichst tiefer Temperatur" in Methylenchlorid oder Benzonitril. Benzonitril erstarrt schon bei −13°C und besitzt bei dieser Temperatur auch kein ausreichendes Lösevermögen mehr. Methylenchlorid läßt sich destillativ nur durch aufwendige Destillation von den niederen Perfluoralkyl-trimethylsilanen abtrennen. Nach einer Ausführungsform der Erfindung wird daher in Gegenwart von polaren, aprotischen Flüssigkeiten gearbeitet, die gegenüber den Reaktionsteilnehmern unter den Reaktionsbedingungen inert sind und deren Siedepunkte ausreichend von denen der herzustellenden Silane verschieden sind, z.B. mindestens 30 oder besser 40°C höher sind. Besonders vorteilhaft ist die Verwendung des technisch gut zugänglichen n-Butyronitrils, das bei tiefen Temperaturen noch gute Lösungseigenschaften besitzt und sich im Siedepunkt ausreichend vom Pentafluoräthyltrimethylsilan unterscheidet.

In der Regel wird die Herstellung des Pentafluoräthyl-trimethylsilans bei Normaldruck vorgenommen, jedoch kann man auch bei erhöhtem oder vermindertem Druck arbeiten, wenn hiermit im allgemeinen auch kein Vorteil verbunden ist. Zweckmäßig arbeitet man unter wasserfreien Bedingungen. Es ist von Vorteil, während der gesamten Dauer der Reaktion, z.B. durch Rühren, für eine gute Durchmischung des Ansatzes zu sorgen. Die Reihenfolge der Vereinigung der drei Komponenten ist nicht kritisch. Die Herstellung kann beispielsweise so erfolgen, daß man das Chlor-trimethylsilan sowie das Pentafluoräthyljodid vorlegt und Phosphorigsäuretrisdiäthylamid zudosiert. Die beiden Reagenzien werden in einer zu Chlor-trimethylsilan äquivalenten Menge oder einem Überschuß, z.B. von bis zu 20 Mol-% eingesetzt.

Als Phosphorigsäuretrisdialkylamide (V) kommen vor allem die niederen Alkylverbindungen in Frage, insbesondere solche mit $C_1$-$C_4$-Alkyl, wie Trisdimethylaminophosphan, Trisdiäthylaminophosphan und Trisdipropyl- bzw. -isopropylaminophosphan ; bevorzugt wird Trisdiäthylaminophosphan $P[N(CH_2CH_3)_2)_3]$ verwendet. Dieses läßt sich sehr einfach in hohen Ausbeuten durch Reaktion von Phosphortrichlorid mit Diäthylamin in einem gegenüber den Reaktionsteilnehmern inerten Lösungsmittel, z.B. einem aliphatischen, cycloaliphatischen oder aromatischen Kohlenwasserstoff oder einem Kohlenwasserstoffgemisch, erzeugen. Die Dialkylaminogruppen können gleiche oder verschiedene Alkylgruppen enthalten.

Da es sich bei dem Pentafluoräthyl-trimethylsilan sowie den bekannten Perfluoralkyl-trimethylsilanen um thermisch stabile (bis 100°C) und gegenüber Luft und Feuchtigkeit beständige Verbindungen handelt, lassen sie sich im Gegensatz zu vielen anderen Perfluororganometallverbindungen sehr einfach für weitere Umsetzungen handhaben.

**Beispiele**

Herstellung von Pentafluoräthyl-trimethylsilan

In einem Rundkolben wurden unter Ausschluß von Feuchtigkeit bei ca. −20°C 43,5 g (0,4 mol) Chlor-trimethylsilan in 100 ml Butyronitril vorgelegt. Es wurden 110 g (0,45 mol) Pentafluoräthyljodid einkondensiert, dann 100,8 g (0,4 mol) Phosphorigsäuretrisdiäthylamid innerhalb von 45 Minuten bei −25°C bis −20°C zudosiert. Anschließend wurde das Reaktionsgemisch unter gutem Rühren 5 Stunden bei ca. −15°C bis −10°C gehalten und dann langsam auf Raumtemperatur gebracht. Das entstandene Silan wurde bei ca. 50 mbar in eine Kältefalle hineinkondensiert. Redestillation des Rohprodukts (79 g) lieferte 66,7 g (87% Ausbeute) an Pentafluoräthyl-trimethylsilan vom Siedepunkt 69-71°C/1013 mbar

| | | | | |
|---|---|---|---|---|
| $C_5H_9F_5Si$ | Ber.: | C 31,24 | H 4,68 | F 49,42 |
| 192,21 | Gef.: | C 31,4 | H 4,8 | F 49,3 |

$^{19}$F-NMR ($CDCl_3$): -82,5 (3F,$CF_3$); -131,8 (2F,$CF_2$)

1) In einem Rundkolben, der mit Rückflußkühler, Innenthermometer und einer Möglichkeit zur Außenkühlung ausgestattet war, wurden 12 g (0,1 mol) Acetophenon in 50 ml Tetraäthylenglykoldimethyläther vorgelegt. Nach Zugabe von 1 g Kaliumfluorid wurden unter gutem Rühren 20,2 g (0,105 mol) Pentafluoräthyl-trimethylsilan zugetropft. Die Reaktionstemperatur wurde zwischen 20 und 30°C gehalten. Nach 6 Stunden war das Keton laut IR-Spektroskopie umgesetzt. Destillation des Reaktionsgemisches lieferte 26,4 g (85% Ausbeute) Silyläther vom Siedepunkt 78-79°C/5 mbar.

```
    CH3
    |
Ph-C-O-Si(CH3)3     Ber.: C 49,99   H 5,49   F 30,41
    |
    CF2CF3          Gef.: C 50,1    H 5,5    F 30,2
```

$^{19}$F-NMR ($CDCl_3$): -78,1 (3F,$CF_3$); -125,5 (2F,$CF_2$)

$^1$H-NMR ($CDCl_3$): 1,92 (s,3H,$CH_3$); 7,3-7,7 (m,5H,Aromat); 0,2 (s,9H, $C\underline{H}_3$-Si)

2) In der im Beispiel 1 beschriebenen Vorrichtung wurden 5,8 g (0,1 mol) Aceton und 14,2 g (0,1 mol) Trifluormethyl-trimethylsilan in 50 ml Tetraäthylenglykoldimethyläther vorgelegt. Nach Zugabe von 1 g KF erfolgte eine spontane Erwärmung des Reaktionsgemisches. Nach Beendigung der exothermen Reaktion wurde das Reaktionsgemisch destilliert, und es wurden 14,6 g (73% Ausbeute) Silyläther vom Siedepunkt (104-105°C/1013 mbar erhalten.

```
     CH3
     |
CH3-C-O-Si(CH3)3    Ber.: C 41,98   H 7,55   F 28,46
     |
     CF3            Gef.: C 41,8    H 7,4    F 28,2
```

$^{19}$F-NMR ($CDCl_3$): - 84,7 (3F,$CF_3$)

$^1$H-NMR ($CDCl_3$): 1,22 (m,6H,$CH_3$); 0,1 (s,9H, $C\underline{H}_3$-Si)

3) In der im Beispiel 1 beschriebenen Vorrichtung wurden 10 g (0,1 mol) Methyl-iso-butylketon und 1 g

Kaliumfluorid in 50 ml Tetraäthylenglykoldimethyläther vorgelegt. Es wurden bei 20 bis 30°C 21,2 g (0,11 mol) Pentafluoräthyl-trimethylsilan unter gutem Rühren zugetropft. Nach 18 Stunden wurden 50 ml Hexan zugetropft ; das Reaktionsgemisch wurde zweimal mit 100 ml Wasser extrahiert und die resultierende organische Phase über $Na_2SO_4$ getrocknet. Bei der Destillation wurden 21,9 g (75% Ausbeute) Silyläther mit dem Siedepunkt 93-94°C/52 mbar erhalten.

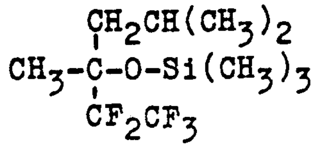

Ber.: C 45,19 H 7,24 F 32,49
Gef.: C 45,3 H 7,1 F 32,2

$^{19}$F-NMR($CDCl_3$): -78,2 (3F,$CF_3$); -123,3 (2F,$CF_2$)
$^{1}$H-NMR ($CDCl_3$): 0,83 (m,6H, $C\underline{H}_3$-CH); 1,29 (s,3H,$CH_3$); 1,43 (m,2H,$CH_2$); 1,6-1,9 (m,1H,CH); 0,1 (s,9H, $C\underline{H}_3$-Si).

4) Bei der Umsetzung von 8,4 g (0,1 mol) Cyclopentanone mit 21,2 g (0,11 mol) Pentafluoräthyl-trimethylsilan analog dem in Beispiel 3 beschriebenen Verfahren erhielt man 18,2 g (66% Ausbeute) Silyläther vom Siedepunkt 88-89°C/52 mbar.

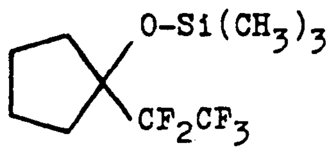

Ber.: C 43,66 H 6,2 F 34,38
Gef.: C 43,3 H 6,1 F 34,5

$^{19}$F-NMR ($CDCl_3$): -79,4 (3F,$CF_3$); -122,9 (2F,$CF_2$)
$^{1}$H-NMR ($CDCl_3$): 1,6 (m,4H,$CH_2$); 1,8 (m,4H,$CH_2$); 0,1 (s,9H, $C\underline{H}_3$-Si).

5) Bei der Umsetzung von 18,2 g (0,1 mol) Benzophenon mit 15,6 g (0,11 mol) Trifluormethyl-trimethylsilan in 80 ml Tetraäthylenglykoldimethyläther analog dem in Beispiel 3 beschriebenen Verfahren erhielt man 26,2 g (81% Ausbeute) Silyläther vom Siedepunkt 98-99°C/0,3 mbar (Fp. : 28-30°C).

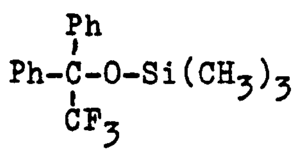

Ber.: C 62,94 H 5,9 F 17,57
Gef.: C 63,2 H 5,9 F 17,3

$^{19}$F-NMR ($CDCl_3$): -73,1 (3F,$CF_3$)
$^{1}$H-NMR ($CDCl_3$): 7,3-7,6 (m,10H, Aromat); 0,1 (s,9H,$CH_3$-Si).

6) Bei der Umsetzung von 16,2 g (0,1 mol) Trifluoracetophenon mit 21,2 g (0,11 mol) Pentafluoräthyl-trimethylsilan analog dem in Beispiel 3 beschriebenen Verfahren erhielt man 26,4 g (85% Ausbeute) Silyläther vom Siedepunkt 89°C/18 mbar.

| | | | |
|---|---|---|---|
| $CF_3$ | | | |
| $Ph-C-O-Si(CH_3)_3$ | Ber.: C 44,06 | H 3,98 | F 42,9 |
| $CF_2CF_3$ | Gef.: C 44,3 | H 3,8 | F 42,3 |

$^{19}F$-NMR ($CDCl_3$): -70,3 (t,3F,$CF_3$); -78,5 (m,3F, $C\underline{F}_3CF_2$); -119,5 (m,2F,$CF_2$)

$^{1}H$-NMR ($CDCl_3$): 7,4-7,7 (m,5H, Aromat); 0,2 (s,9H, $C\underline{H}_3$-Si).

7) Bei der Umsetzung von 5,6 g (0,1 mol) Acrolein mit 15,6 g (0,11 mol) Trifluormethyl-trimethylsilan analog dem in Beispiel 3 beschriebenen Verfahren erhielt man 14,8 g (75% Ausbeute) Silyläther vom Siedepunkt 53-54°C/100 mbar.

| | | | |
|---|---|---|---|
| $CH=CH_2$ | | | |
| $H-C-O-Si(CH_3)_3$ | Ber.: C 42,4 | H 6,61 | F 28,75 |
| $CF_3$ | Gef.: C 42,5 | H 6,8 | F 29,1 |

$^{19}F$-NMR ($CDCl_3$): -79,52 (d,3F,$CF_3$)

$^{1}H$-NMR ($CDCl_3$): 4,46 (m,1H, $C\underline{H}-CF_3$); 5,4-6,2 (m,3H, $CH=CH_2$) 0,1 (s,9H, $C\underline{H}_3$-Si),

8) Bei der Umsetzung von 10,6 g (0,1 mol) Benzaldehyd mit 26,6 g (0,11 mol) Heptafluor-iso-propyl-trimethylsilan analog dem in Beispiel 3 beschriebenen Verfahren erhielt man 23,3 g (67% Ausbeute) Silyläther vom Siedepunkt 107-109°C/35 mbar.

| | | | |
|---|---|---|---|
| Ph | | | |
| $H-C-O-Si(CH_3)_3$ | Ber.: C 44,82 | H 4,34 | F 38,18 |
| $CF(CF_3)_2$ | Gef.: C 44,6 | H 4,3 | F 38,4 |

$^{19}F$-NMR ($CDCl_3$): -72,9 (m,F,$CF_3$); -180,3 (m,1F,CF)

$^{1}H$-NMR ($CDCl_3$): 5,2 (m,1H,CH); 7,2-7,5 (m,5H, Aromat); 0,1 (s,9H, $C\underline{H}_3$-Si).

9) In einem Rundkolben wurden 10,6 g (0,1 mol) Benzaldehyd und 1 g Kaliumfluorid in 50 ml Tetraäthylengylkoldimethyläther vorgelegt. Bei 20°C bis 30°C wurden 15,6 g (0,11 mol) Trifluormethyltrimethylsilan unter gutem Rühren zugetropft. Nach weiteren 5 Stunden bei dieser Temperatur wurde analog Beispiel 3 aufgearbeitet. Bei der Destillation des Rohprodukts erhielt man 23,3 g (90% Ausbeute) Silyläther vom Siedepunkt 63-64°C/6 mbar.

| | | | |
|---|---|---|---|
| $H-\overset{Ph}{\underset{CF_3}{C}}-O-Si(CH_3)_3$ | Ber.: C 53,2 | H 6,09 | F 22,96 |
| | Gef.: C 53,3 | H 6,1 | F 22,8 |

$^{19}F$-NMR ($CDCl_3$): -78,6 (d, J= 6Hz (F-H), 3F, $CF_3$)
$^{1}H$-NMR ($CDCl_3$): 4,81 (q, J= 6Hz, (H-$CF_3$), 1H,CH), 7,2-7,4 (m,5H, Aromat); 0,1 (s,9H, $CH_3$-Si)

10) Benzaldehyd wurde wie im Beispiel 9 mit Trifluormethyl-trimethylsilan umgesetzt. Nach einer Reaktionszeit von 5 Stunden wurden 20 ml Wasser zugesetzt, das Reaktionsgemisch kurz auf 80°C erhitzt. Anschließend wurden 50 ml Hexan zugesetzt. Das Reaktionsgemisch wurde dann zweimal mit 100 ml Wasser extrahiert und die resultierende organische Phase über $Na_2SO_4$ getrocknet. Bei der Destillation des Rohproduktes erhielt man 15,4 g (88% Ausbeute) 1-Phenyl-2,2,2-trifluoräthanol (Siedepunkt 68-69°C/6 mbar).

**Patentansprüche**

1. Verfahren zur Herstellung von Perfluoralkylgruppen enthaltenden Silyläthern und Alkoholen dadurch gekennzeichnet, daß man ein Perfluoralkyl-trimethylsilan der allgemeinen Formel

$$(CH_3)_3Si\text{-}C_nF_{2n+1} \quad (I),$$

worin der Rest $C_nF_{2n+1}$ einen Perfluoralkylrest mit n = 1 bis n = 6 bedeutet, mit einer Carbonylverbindung der allgemeinen Formel

$$R^1\text{-}CO\text{-}R^2 \quad (II),$$

worin $R^1$ einen Kohlenwasserstoffrest oder Wasserstoff und $R^2$ einen Kohlenwasserstoff-, Perfluoralkyl- oder Perfluorarylrest bedeutet, $R^1$ und $R^2$ zusammen aber auch Bestandteil eines alicyclischen Ringsystems sein können, in Gegenwart eines salzartigen Fluorids, das wenigstens teilweise im Reaktionsmedium löslich ist, als Katalysator zu Silyläthern der Formel

$$R^1 - \overset{R^2}{\underset{C_nF_{2n+1}}{C}} - O - Si(CH_3)_3 \qquad (III)$$

umsetzt und diese isoliert oder durch Hydrolyse in Alkohole der Formel

$$R^1 - \overset{R^2}{\underset{C_nF_{2n+1}}{C}} - OH \qquad (IV)$$

überführt.

2. Verfahren nach Anspruch 1, dadurch gekennzeichnet, daß ein Alkalifluorid oder Alkalibifluorid als Katalysator verwendet wird.

3. Verfahren nach Anspruch 1 oder 2, dadurch gekennzeichnet, daß der Katalysator in einer Menge von 2 bis 30, vorzugsweise 10 bis 25 Mol-%, bezogen auf die Carbonylverbindung verwendet wird.

4. Verfahren nach einem oder mehreren der Ansprüche 1 bis 3, dadurch gekennzeichnet, daß die Umsetzung unter wasserfreien Bedingungen in Gegenwart eines gegenüber den Reaktionsteilnehmern unter den Reaktionsbedingungen inerten Lösungs- oder Verdünnungsmittels durchgeführt wird, das ein Lösungsvermögen für den Katalysator hat und vorzugsweise ein Äther oder Dimethylformamid ist.

5. Verfahren nach einem oder mehreren der Ansprüche 1 bis 4, dadurch gekennzeichnet, daß die Umsetzung bei Temperaturen von etwa 0°C bis 100°C, vorzugsweise 20 bis 60°C durchgeführt wird.

6. Verfahren nach einem oder mehreren der Ansprüche 1 bis 5, dadurch gekennzeichnet, daß das Silan in einer zur Carbonylverbindung mindestens äquivalenten Menge und höchstens in einem Überschuß von bis zu 25 Mol-% eingesetzt wird.

7. Verfahren nach einem oder mehreren der Ansprüche 1 bis 6, dadurch gekennzeichnet, daß die Reste $R^1$ und $R^2$ jeweils bis zu 10, insbesondere bis zu 6 C-Atome haben.

## Claims

1. A process for the preparation of alcohols and silyl ethers containing perfluoralkyl groups, which comprises reacting a perfluoroalkyltrimethylsilane of the general formula

$$(CH_3)_3Si\text{-}C_nF_{2n+1} \quad (I)$$

in which the $C_nF_{2n+1}$ radical denotes a perfluoroalkyl radical where n = 1 to n = 6, with a carbonyl compound of the general formula

$$R^1\text{-CO-}R^2 \quad (II)$$

in which $R^1$ denotes a hydrocarbon radical or hydrogen and $R^2$ denotes a hydrocarbon, perfluoroalkyl or perfluoroaryl radical, or $R^1$ and $R^2$ together can also be part of an alicyclic ring system, in the presence of a salt-like fluoride which is at least partially soluble in the reaction mixture, as catalyst to give silyl ethers of the formula

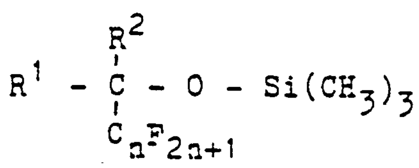

(III)

and isolating or converting the latter by hydrolysis into alcohols of the formula

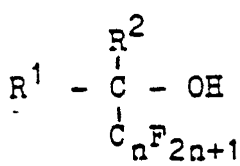

(IV)

2. The process as claimed in claim 1, wherein an alkali fluoride or alkali hydrogen fluoride is used as the catalyst.

3. The process as claimed in claim 1 or 2, wherein the catalyst is used in an amount of from 2 to 30, preferably 10 to 25 mol-%, based on the carbonyl compound.

4. The process as claimed in one or more of claims 1 to 3, wherein the reaction is carried out under anhydrous conditions in the presence of a solvent or diluent which has a solution capacity for the catalyst, is inert towards the reactants under the reaction conditions and is preferably an ether or dimethyl formamide.

5. The process as claimed in one or more of claims 1 to 4, wherein the reaction is carried out at temperatures of from about 0°C to 100°C, preferably 20 to 60°C.

6. The process as claimed in one or more of claims 1 to 5, wherein the silane is employed in an amount at least equivalent to the amount of the carbonyl compound and at most in an excess of up to 25 mol-%.

7. The process as claimed in one or more of claims 1 to 6, wherein each of the radicals $R^1$ and $R^2$ has up to 10, especially up to 6, carbon atoms.

**Revendications**

1. Procédé pour préparer des éthers silyliques et des alcools contenant des radicaux perfluoralkyles, procédé caractérisé en ce qu'on fait réagir un perfluoralkyl-triméthylsilane répondant à la formule générale I :

$$(CH_3)_3Si\text{-}C_nF_{2n+1} \quad \text{(I)}$$

dans laquelle le radical $—C_nF_{2n+1}$ est un radical perfluoralkyle et n désigne un nombre de 1 à 6, avec un composé carbonylique répondant à la formule générale II :

$$R^1\text{-CO-}R^2 \quad \text{(II)}$$

dans laquelle $R^1$ représente un radical hydrocarboné ou un atome d'hydrogène et $R^2$ représente un radical hydrocarboné, un radical perfluoralkyle ou un radical perfluoraryle, ou encore $R^1$ et $R^2$ peuvent également former ensemble une partie d'un système alicyclique, en présence d'un catalyseur qui est un fluorure de nature saline au moins partiellement soluble dans le milieu réactionnel, réaction qui conduit à des éthers silyliques de formule III :

$$R^1-\overset{\displaystyle R^2}{\underset{\displaystyle C_nF_{2n+1}}{C}} - O - Si(CH_3)_3 \qquad \text{(III)},$$

et on isole ceux-ci ou on les transforme, par hydrolyse, en alcools de formule IV :

$$R^1-\overset{\displaystyle R^2}{\underset{\displaystyle C_nF_{2n+1}}{C}} - OH \qquad \text{(IV)}.$$

2. Procédé selon la revendication 1 caractérisé en ce qu'on utilise un fluorure alcalin ou un bifluorure alcalin comme catalyseur.

3. Procédé selon l'une des revendications 1 et 2, caractérisé en ce que le catalyseur est mis en jeu en une quantité de 2 à 30% en moles, de préférence de 10 à 25% en moles, par rapport au composé carbonylique.

4. Procédé selon l'une quelconque des revendications 1 à 3, caractérisé en ce que la réaction est effectuée dans des conditions anhydres, en présence d'un solvant ou diluant qui, dans les conditions de la réaction, est inerte à l'égard des partenaires réactionnels et qui a un pouvoir solvant pour le catalyseur, ce solvant ou diluant étant de préférence un éther ou le diméthylformamide.

5. Procédé selon l'une quelconque des revendications 1 à 4, caractérisé en ce que la réaction est effectuée à des températures d'environ 0 à 100°C, de préférence de 20 à 60°C.

6. Procédé selon l'une quelconque des revendications 1 à 5, caractérisé en ce que le silane est mis en jeu en une quantité au moins équivalente par rapport au composé carbonylique et au plus en un excès inférieur ou égal à 25% en moles.

7. Procédé selon l'une quelconque des revendications 1 à 6, caractérisé en ce que les radicaux $R^1$ et $R^2$ contiennent chacun au plus 10 atomes de carbone, plus particulièrement au plus 6.